# FASCICULE DE BREVET EUROPEEN

(11) **EP 3 449 876 B1**
(45) Date de publication et mention de la délivrance du brevet: **15.04.2020**
(21) Numéro de dépôt: 18192345.9
(22) Date de dépôt: 04.09.2018
(51) Int. Cl.: A61F 2/42

(54) **PROTHÈSE DE CHEVILLE AMÉLIORÉE**
VERBESSERTE KNÖCHELPROTHESE
IMPROVED ANKLE PROSTHESIS

(30) Priorité: 05.09.2017 FR 1758185
(43) Date de publication de la demande: 06.03.2019
(73) Titulaire: In2Bones, 69130 Ecully (FR)
(72) Inventeur: LEEMRIJSE, Thibaut, Jean, Pierre, Henry, 1200 Bruxelles (BE); PUTZEYS, Pit, L8119 Bridel (LU); PAUL, Laurent, François, René, 1495 Mellery (BE); AGREN, Per-Henrik, 11140 Stockholm (SE); BESSE, Jean-Luc, Pierre, Marie, 69970 Chaponnay (FR)
(74) Mandataire: Weber, Jean-François

(56) Documents cités:
- WO-A1-2014/149952
- FR-A1- 2 905 259
- US-A1- 2014 180 427

## Description

L'invention se rapporte au domaine général des prothèses de cheville, c'est-à-dire des dispositifs implantables destinés au remplacement d'articulations de chevilles, en particulier dans le cadre d'un traitement orthopédique.

L'invention concerne plus précisément une prothèse de cheville, comprenant un composant talaire qui comporte une face supérieure talaire définissant une première surface articulaire et qui s'étend entre un bord antérieur talaire et un bord postérieur talaire opposé selon une première direction moyenne, ladite première surface articulaire étant incurvée selon ladite première direction moyenne.

Afin de traiter certaines pathologies osseuses de la cheville, telle que l'arthrose, entraînant une dégradation ou une disparition du cartilage articulaire, il est connu de procéder à une arthrodèse de l'articulation tibio-tarsienne. Une telle opération d'arthrodèse vise à limiter, voire bloquer totalement, la mobilité de cheville par ostéosynthèse, afin de faire cesser la douleur articulaire ressentie par le patient. Si l'arthrodèse de l'articulation tibio-tarsienne donne généralement satisfaction, son principal inconvénient réside précisément dans la suppression de la mobilité de l'articulation, qui doit alors être compensée autant que de possible par les autres articulations de la jambe du patient. Une longue période d'adaptation est alors nécessaire au patient pour retrouver, après opération, des capacités de locomotion satisfaisantes. En outre, le blocage de l'articulation tibio-tarsienne engendre des contraintes mécaniques élevées sur les articulations voisines, lesquelles sont alors exposées à un risque élevé de dégénérescence précoce.

C'est la raison pour laquelle il a été proposé de procéder, dans certains cas de figures, à une arthroplastie de la cheville comme alternative à l'arthrodèse, c'est-à-dire au remplacement de l'articulation endommagée de la cheville par une articulation prothétique, artificielle. Il a ainsi été introduit une prothèse de cheville formée de plusieurs composants, à savoir un composant talaire et un composant tibial, respectivement destinés à être rapportés au talus et au tibia, et un patin en plastique qui est, quant à lui, prévu pour être intercalé entre le composant talaire et le composant tibial et venir s'articuler en contact avec le composant talaire. Le recours à une telle prothèse connue permet, contrairement à l'arthrodèse classique, de conserver une bonne mobilité de la cheville du patient, facilitant ainsi la marche, et de préserver les différentes articulations du pied et de la jambe du patient. Il a cependant été observé qu'une telle prothèse de cheville connue reste encore perfectible, en particulier en matière de reproductibilité des mouvements naturels complexes de la cheville et de leur amplitude.

Le document WO 2014/149952 décrit une prothèse de cheville selon le préambule de la revendication 1 de la présente invention.

Les objets assignés à la présente invention visent en conséquence à remédier aux différents inconvénients énumérés précédemment et à proposer une nouvelle prothèse de cheville présentant une cinématique améliorée, plus respectueuse des mouvements naturels de la cheville, et favorisant un retour pour le patient à une locomotion aisée et confortable.

Un autre objet de l'invention vise à proposer une nouvelle prothèse de cheville dont la mise en place est à la fois rapide, facile et particulièrement peu traumatisante pour le patient.

Un autre objet de l'invention vise à proposer une nouvelle prothèse de cheville, robuste et résistante, et dont la tenue dans le corps du patient est particulièrement fiable.

Un autre objet de l'invention vise à proposer une nouvelle prothèse de cheville dont la fabrication est relativement aisée.

Un autre objet de l'invention vise à proposer une nouvelle prothèse de cheville permettant de réduire le coût de l'intervention chirurgicale de pose de la prothèse.

Un autre objet de l'invention vise à proposer une nouvelle prothèse de cheville permettant de réduire le risque pour la santé du patient lié à la pose de la prothèse et à la thérapie.

Un autre objet de l'invention vise à proposer une nouvelle prothèse de cheville permettant de traiter une pathologie osseuse du patient de façon particulièrement efficace et rapide.

Les objets assignés à l'invention sont atteints à l'aide d'une prothèse de cheville, comprenant un composant talaire qui comporte une face supérieure talaire définissant une première surface articulaire et qui s'étend entre un bord antérieur talaire et un bord postérieur talaire opposé selon une première direction moyenne, ladite première surface articulaire étant incurvée selon ladite première direction moyenne, ladite première surface articulaire comprenant une première partie courbe et une deuxième partie courbe, qui s'étendent chacune selon ladite première direction moyenne, ladite première partie courbe présentant une première courbure et ladite deuxième partie courbe présentant une deuxième courbure, ladite prothèse étant caractérisée en ce que lesdites première et deuxième parties courbes définissent respectivement une portion antérieure et une portion postérieure de ladite première surface articulaire, ladite première courbure étant supérieure à ladite deuxième courbure.

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :

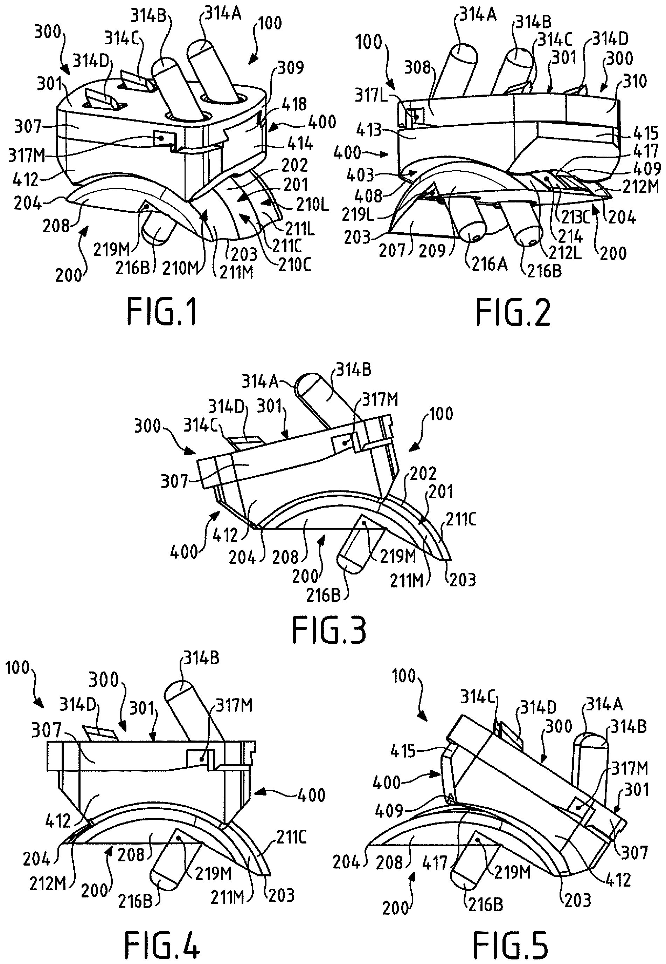

- les figures 1 et 2 illustrent, selon des vues en perspective différentes, un mode de réalisation préférentiel de la prothèse selon l'invention dans lequel cette dernière comprend, outre un composant talaire, un composant tibial et un composant intermédiaire ;
- les figures 3 à 5, illustrent, selon des vues médiales, trois configurations spatiales que la prothèse des figures 1 et 2 est susceptible d'adopter selon l'orientation relative des pied et tibia gauches d'un patient, à savoir respectivement à une position de flexion plantaire (figure 3), une position dite neutre (figure 4) et une position de flexion dorsale (figure 5) ;

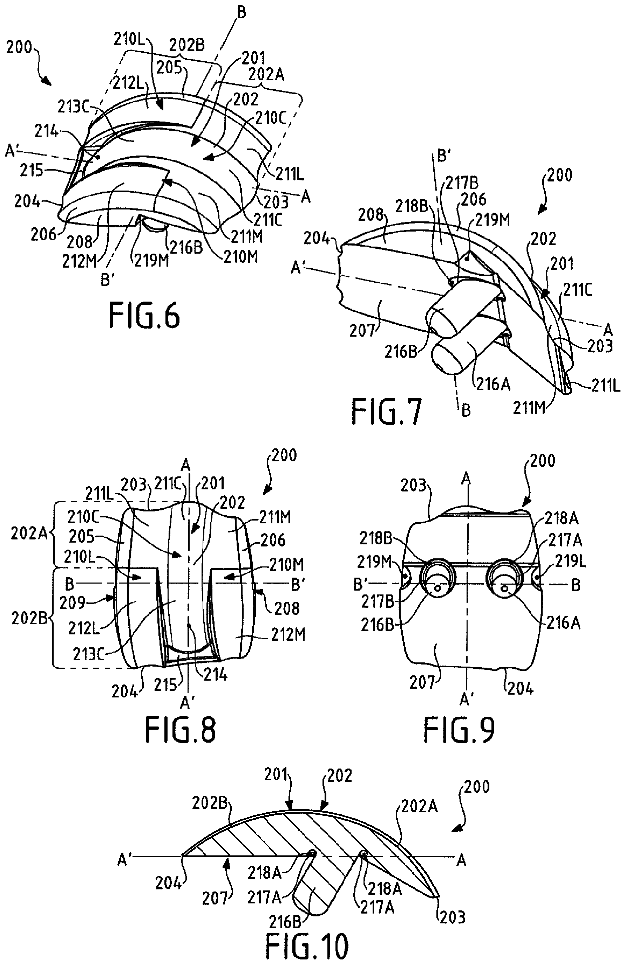

- les figures 6 et 7 illustrent, respectivement selon des vues en perspective de dessus et de dessous, le composant talaire de la prothèse des figures 1 et 2. Les figures 8 et 9 illustrent, respectivement en vue de dessus et en vue de dessous, le composant talaire des figures 6 et 7. La figure 10 illustre, selon une vue en coupe antéro-postérieure, le composant talaire des figures 6 à 9 ;

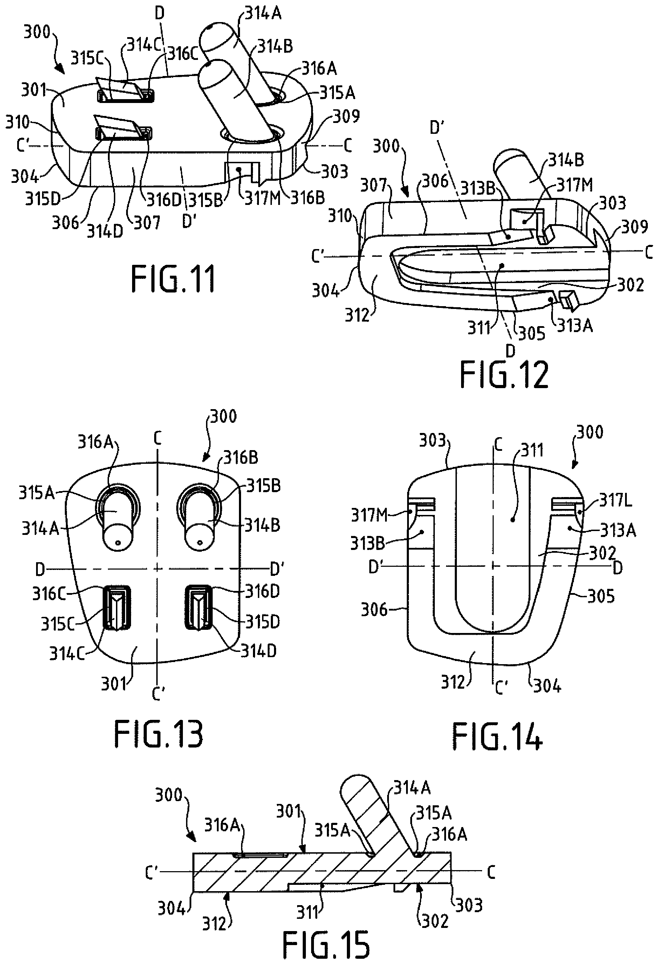

- les figures 11 et 12 illustrent, respectivement selon des vues en perspective de dessus et de dessous, le composant tibial de la prothèse des figures 1 et 2. Les figures 13 et 14 illustrent, respectivement en vue de dessus et en vue de dessous, le composant tibial des figures 11 et 12. La figure 15 illustre, selon une vue en coupe antéro-postérieure, le composant tibial des figures 11 à 14 ;

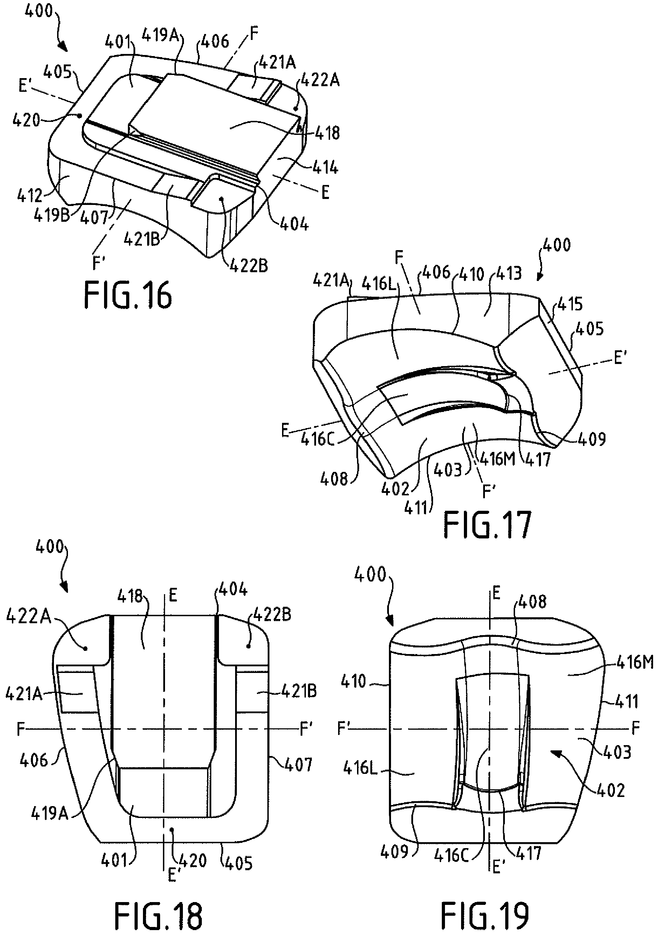

- les figures 16 et 17 illustrent, respectivement selon des vues en perspective de dessus et de dessous, le composant intermédiaire de la prothèse des figures 1 et 2. Les figures 18 et 19 illustrent, respectivement, en vue de dessus et de dessous, le composant intermédiaire des figures 16 et 17.

L'invention concerne une prothèse de cheville 100, dont un mode de réalisation préférentiel est illustré aux figures 1 et 2. Ladite prothèse 100 constitue un dispositif implantable par voie chirurgicale dans le corps d'un patient, humain ou animal, qui est destiné au remplacement d'une articulation tibio-tarsienne donnée. Avantageusement, ladite prothèse 100 est conçue pour remplacer totalement l'articulation tibio-tarsienne concernée (prothèse totale de cheville, PTC). A ce titre, la prothèse 100 selon l'invention est conçue pour être insérée et intercalée entre une extrémité inférieure d'un tibia et un talus correspondant d'un pied du patient. Avantageusement, le tibia et le talus considérés auront fait l'objet, préalablement à la mise en place de la prothèse 100 selon l'invention dans le corps du patient, d'une préparation adéquate, et par exemple d'une extraction d'éléments cartilagineux et de coupes osseuses, de sorte à supprimer tout ou partie des surfaces articulaires naturelles de l'articulation tibio-tarsienne à remplacer. La prothèse 100 illustrée en exemple aux figures 1 à 5 est destinée à être mise en place au niveau d'un pied gauche d'un patient. Bien évidemment, l'invention recouvre également une prothèse qui serait destinée à être mise en place au niveau d'un pied droit du patient. Avantageusement, celle-ci serait définie par symétrie, par rapport au plan sagittal du patient, de la prothèse 100 illustrée aux figures.

Conformément à l'invention, ladite prothèse 100 comprend un composant talaire 200, dont un mode de réalisation préférentiel est illustré aux figures 6 à 10, et qui est avantageusement destiné à être rapporté au talus d'un pied du patient. Le composant talaire 200 comporte une face supérieure talaire 201, qui définit une première surface articulaire 202 de la prothèse 100. Tel qu'illustré, ladite face supérieure talaire 201 s'étend entre un bord antérieur talaire 203 et un bord postérieur talaire 204 opposé selon une première direction A-A' moyenne et, de préférence, entre un bord latéral talaire 205 et un bord médial talaire 206 opposé selon une deuxième direction B-B' moyenne, orthogonale à ladite première direction A-A' moyenne. Avantageusement, ladite première direction A-A' moyenne pourra correspondre à une première direction antéro-postérieure moyenne en usage normal de la prothèse 100. Ladite deuxième direction B-B' moyenne pourra alors avantageusement correspondre à une première direction latéro-médiale moyenne. Avantageusement, ledit bord latéral talaire 205 et ledit bord médial talaire 206 sont arrondis, chanfreinés.

Il est ici à noter que les termes « *postérieur* », « *antérieur* », « *médial* » et « *latéral* » sont préférentiellement utilisés dans la présente description pour qualifier des éléments ou des caractéristiques en lien avec leur orientation respective relativement au corps du patient, en usage normal de la prothèse 100. Ainsi, le terme *« médial »* est préférentiellement utilisé pour désigner un élément de la prothèse 100 qui destiné à être positionné et orienté du côté le plus proche de l'axe medio-sagittal (ou axe médian) du corps du patient, autrement dit le côté orienté vers l'intérieur du pied et de la jambe du patient. Par opposition, le terme « *latéral* » est utilisé en relation avec le côté le plus éloigné de l'axe medio-sagittal. Suivant la même logique, les termes « *postérieur* » et « *antérieur* » renvoient de préférence respectivement à un positionnement vers l'arrière, respectivement vers l'avant, relativement au plan frontal du patient.

Selon le mode de réalisation préférentiel illustré aux figures, le composant talaire 200 comporte également une face inférieure talaire 207, opposée à ladite face supérieure talaire 201, et qui est avantageusement reliée à cette dernière au niveau desdits bord antérieur talaire 203 et bord postérieur talaire 204. Ladite face inférieure talaire 207 est préférentiellement destinée à être positionnée en contact dans ou sur une zone du talus (ou astragale) du patient spécialement apprêtée au préalable. De préférence, la face inférieure talaire 207 est définie par des première et deuxième portions contigües sensiblement planes (figure 7), qui s'étendent globalement selon des plans respectifs sécants, par exemple selon un angle d'élévation de 30° de la première portion plane relativement à la deuxième portion plane. La première portion plane forme préférentiellement une portion antérieure de la face inférieure talaire 207, tandis que la deuxième portion plane, qui s'étend selon un plan sensiblement horizontal en usage normal de la prothèse 100 selon l'invention, forme avantageusement une portion à la fois centrale et postérieure de la face inférieure talaire 207. Un congé peut être prévu à la jonction desdites première et deuxième portions planes. Une telle conception de la face inférieure talaire 207, particulièrement simple, limite le nombre et la complexité des coupes osseuses nécessaires (en l'espèce, deux) à la préparation préalable du talus et facilite la mise en place du composant talaire 200 dans le corps du patient. Tel qu'illustré, le composant talaire 200 peut également comporter une face médiale talaire 208 et une face latérale talaire 209 opposée, lesquelles relient respectivement le bord médial talaire 206 et le bord latéral talaire 205 à ladite face inférieure talaire 207. De préférence, lesdites face médiale talaire 208 et face latérale talaire 209 sont globalement bombées vers l'extérieur du composant talaire 200, afin de respecter au mieux la conformation anatomique naturelle de la zone du talus au niveau de laquelle le composant talaire 200 est destiné à être mis en place.

De préférence, la prothèse 100 selon l'invention comprend un composant tibial 300 (figures 11 à 15), destiné à être rapporté à une extrémité inférieure du tibia du patient. Ledit composant tibial 300 comporte une face supérieure tibiale 301 et une face inférieure tibiale 302 opposée. Tel qu'illustré, ladite face inférieure tibiale 302 s'étend préférentiellement, d'une part, entre un bord antérieur tibial 303 et un bord postérieur tibial 304 opposé, de préférence selon une troisième direction C-C' moyenne et, d'autre part, entre un bord latéral tibial 305 et un bord médial tibial 306 opposé, de préférence selon une quatrième direction D-D' moyenne, orthogonale à ladite troisième direction C-C' moyenne. Avantageusement, ladite troisième direction C-C' moyenne pourra correspondre à une deuxième direction antéro-postérieure moyenne en usage normal de la prothèse 100. Ladite quatrième direction D-D' moyenne pourra alors avantageusement correspondre à une deuxième direction latéro-médiale moyenne. Lesdites deuxième direction antéro-postérieure moyenne et deuxième direction latéro-médiale moyenne du composant tibial 300 sont avantageusement respectivement parallèles auxdites première direction antéro-postérieure moyenne et première direction latéro-médiale moyenne du composant talaire 200, en usage normal de la prothèse 100.

Dans le mode de réalisation préférentiel illustré aux figures, la face supérieure tibiale 301 et la face inférieure tibiale 302 s'étendent respectivement selon des plans d'extension moyens sensiblement parallèles, ledit composant tibial 300 se présentant sous la forme générale d'une plaque. Ladite face supérieure tibiale 301 est préférentiellement destinée à être positionnée en contact dans ou sur une zone du tibia spécialement apprêtée au préalable. Tel qu'illustré, le composant tibial 300 peut également comporter une face médiale tibiale 307 et une face latérale tibiale 308 opposée, lesquelles relient respectivement le bord médial tibial 306 et le bord latéral tibial 305 à ladite face supérieure tibiale 301. Avantageusement, ledit bord médial tibial 306 est sensiblement rectiligne et ladite face médiale tibiale 307 est sensiblement plane (figures 11 à 14), de sorte à ce que le chirurgien puisse positionner précisément ledit composant tibial 300 dans le corps du patient, en alignant ladite face médiale tibiale 307 le long d'une coupe rectiligne réalisée au niveau de la malléole interne. Cela permet de réduire avantageusement les zones de coupes osseuses non recouvertes, qui pourraient favoriser l'apparition de géodes ou de kystes. En outre, le composant tibial 300 peut comporter une face antérieure tibiale 309 et une face postérieure tibiale 310 opposée, lesquelles relient quant à elles respectivement le bord antérieur tibial 303 et le bord postérieur tibial 304 à ladite face supérieure tibiale 301.

De préférence, ledit composant talaire 200 et/ou ledit composant tibial 300 forment respectivement une pièce monobloc d'un matériau biocompatible et résistant à l'usure. Avantageusement, ledit composant talaire 200 et / ou ledit composant tibial 300 sont en un matériau métallique, par exemple en alliage chrome-cobalt CrCo, en acier inoxydable, ou encore en titane. Selon une variante, ledit composant talaire 200 et ledit composant tibial 300 sont respectivement une pièce de fonderie. Selon une variante alternative, ledit composant talaire 200 et ledit composant tibial 300 sont respectivement une pièce usinée. Bien évidemment, d'autres matériaux convenables pourront être envisagés, tel que par exemple un matériau céramique, ainsi que d'autres mode de fabrication (par injection, moulage, frittage, etc.).

De manière avantageuse, la face inférieure talaire 207 du composant talaire 200 et / ou la face supérieure tibiale 301 du composant tibial 300 pourront être pourvues d'un revêtement de surface particulier (par exemple en titane poreux ou en hydroxyapatite), ou avoir fait l'objet d'un traitement mécanique particulier (sablage, rainurage, etc.), afin de favoriser l'accroche osseuse du composant talaire 200 au talus et /ou du composant tibial 300 au tibia correspondant.

De préférence, la prothèse 100 selon l'invention comprend également un composant intermédiaire 400 (ou patin, ou encore insert) (figures 16 à 19), qui est conçu pour être intercalé entre ledit composant talaire 200 et ledit composant tibial 300, comme illustré en exemple aux figures 1 à 5. Ledit composant intermédiaire 400 comprend une face supérieure intermédiaire 401, préférentiellement destinée à venir en contact avec la face inférieure tibiale 302 du composant tibial 300, et une face inférieure intermédiaire 402 opposée, qui est quant à elle préférentiellement destinée à venir en contact avec la face supérieure talaire 201 du composant talaire 200. Ladite face inférieure intermédiaire 402 définit avantageusement une deuxième surface articulaire 403 de la prothèse 100, conçue pour coopérer avec ladite première surface articulaire 202 définie par la face supérieure talaire 201 du composant talaire 200.

De préférence, ladite face supérieure intermédiaire 401 s'étend d'une part entre un premier bord antérieur intermédiaire 404 et un premier bord postérieur intermédiaire 405 opposé (par exemple selon une cinquième direction E-E' moyenne) et, d'autre part, entre un premier bord latéral intermédiaire 406 et un premier bord médial intermédiaire 407 opposé (par exemple selon une sixième direction F-F' moyenne, orthogonale à ladite cinquième direction E-E' moyenne). Ladite face inférieure intermédiaire 402 s'étend quant à elle préférentiellement d'une part entre un deuxième bord antérieur intermédiaire 408 et un deuxième bord postérieur intermédiaire 409 opposé, selon ladite cinquième direction E-E' moyenne et, d'autre part, entre un deuxième bord latéral intermédiaire 410 et un deuxième bord médial intermédiaire 411 opposé, selon ladite sixième direction F-F' moyenne. Ledit deuxième bord antérieur intermédiaire 408 et ledit deuxième bord postérieur intermédiaire 409 peuvent être avantageusement chanfreinés, tel qu'illustré, pour limiter les risques d'irritation ou d'endommagement des tissus mous environnants, en usage de la prothèse 100. Le composant intermédiaire 400 peut également comporter une face médiale intermédiaire 412 et une face latérale intermédiaire 413 opposée, lesquelles relient respectivement le premier bord médial intermédiaire 407 et le premier bord latéral intermédiaire 406 à ladite face inférieure intermédiaire 402. Avantageusement, la face médiale intermédiaire 412 est sensiblement plane, pour des raisons sensiblement identiques à celles exposées ci-avant en lien avec la face médiale tibiale 307 du composant tibial 300, tandis que la face latérale intermédiaire 413 peut être globalement bombée, avec une concavité orientée en direction de ladite face médiale intermédiaire 412. En outre, le composant intermédiaire 400 peut comporter une face antérieure intermédiaire 414 et une face postérieure intermédiaire 415 opposée, lesquelles relient quant à elles respectivement le premier bord antérieur intermédiaire 404 et le premier bord postérieur intermédiaire 405 à ladite face inférieure intermédiaire 402.

Tel qu'illustré aux figures 3 à 5, le composant intermédiaire 400 est susceptible de se déplacer en glissant en contact sur le composant talaire 200 suivant une direction antéro-postérieure moyenne et, en particulier, selon une course globalement postéro-antérieure (c'est-à-dire de l'arrière vers l'avant) entre le bord postérieur talaire 204 (flexion plantaire, figure 3) et le bord antérieur talaire 203 (flexion dorsale, figure 5), en passant par une position intérimaire dite neutre (figure 4), selon l'inclinaison affectée par le pied du patient par rapport à son tibia.

De préférence, ledit composant intermédiaire 400 est une pièce monobloc en un matériau présentant un faible coefficient de frottement, par exemple un matériau plastique tel qu'un polyéthylène haute densité (PEHD). Il peut s'agir, par exemple, d'une pièce usinée ou moulée.

Alternativement, la prothèse 100 pourrait ne pas comprendre de composant intermédiaire 400, la face inférieure tibiale 302 pouvant alors être conçue pour définir par elle-même une deuxième surface articulaire destinée à coopérer directement avec la première surface articulaire 202 définie par la face supérieure talaire 201 du composant talaire 200.

Selon l'invention, et tel que cela ressort notamment de la figure 6, ladite première surface articulaire 202 est incurvée, bombée, selon ladite première direction A-A' moyenne. Réciproquement, la deuxième surface articulaire 403 définie par la face inférieure intermédiaire 402 du composant talaire 400 est préférentiellement incurvée selon ladite cinquième direction E-E' moyenne. Ladite première surface articulaire 202 est de préférence globalement convexe (c'est-à-dire avec une concavité orientée en direction de la face inférieure talaire 207 du composant talaire 200), tandis que la deuxième surface articulaire 403 est globalement concave. Selon une variante de réalisation alternative (non illustrée), une configuration inversée pourrait être envisagée sans pour autant sortir du cadre de l'invention, la première surface articulaire 202 étant globalement concave, tandis que la deuxième surface articulaire 403 est globalement convexe.

Avantageusement, ladite première surface articulaire 202 (et, de préférence, ladite deuxième surface articulaire 403) présente(nt) plus spécifiquement (chacune) la forme générale moyenne d'une fraction de surface (fictive) sensiblement tronconique, issue d'un cône fictif, laquelle surface est préférentiellement orientée pour que sa grande base soit orientée en direction de la malléole externe et que sa petite base soit orientée en direction de la malléole interne du pied considéré. Ainsi, lorsque le composant intermédiaire 400 se déplace en contact de frottement relativement au composant talaire 200, par coopération desdites première 202 et deuxième 403 surfaces articulaires, le composant intermédiaire 400 ne décrit pas une trajectoire strictement antéro-postérieure ou postéro-antérieure, mais au contraire une trajectoire plus ou moins courbe. Le pied du patient est ainsi avantageusement guidé sur le côté latéral (sens médio-latéral) en flexion dorsale et, inversement, sur le côté médial (sens latéro-médial) en flexion plantaire. On reproduit ainsi au mieux la cinématique physiologique naturelle de l'articulation de la cheville. Alternativement, lesdites première 202 et deuxième 403 surfaces articulaires pourraient présenter chacune la forme générale moyenne d'une fraction de surface (fictive) cylindrique, de sorte à définir au contraire une cinématique articulaire dans laquelle le composant intermédiaire 400 se déplace selon une trajectoire sensiblement antéro-postérieure ou postéro-antérieure.

Selon l'invention, ladite première surface articulaire 202 comprend au moins deux parties surfaciques courbes distinctes, à savoir au moins une première partie courbe 202A et une deuxième partie courbe 202B, qui s'étendent chacune selon ladite première direction A-A' moyenne, alignées l'une derrière l'autre selon ladite première direction A-A' moyenne, ladite première partie courbe 202A présentant une première courbure et ladite deuxième partie courbe 202B présentant une deuxième courbure, selon ladite première direction A-A' moyenne. Ainsi, lorsqu'elle est vue en coupe dans plan parallèle à ladite première direction A-A' moyenne et orthogonal à ladite deuxième direction B-B' moyenne (figure 10), ladite première surface articulaire 202 est décrite par au moins deux portions curvilignes distinctes (respectivement désignées sur la figure 10 par les mêmes références 202A et 202B que pour les première et deuxième parties courbes correspondantes), formant chacune de préférence un arc dont la concavité est préférentiellement dirigée en direction de la face inférieure talaire 207, lesdites portions curvilignes ayant des centres de courbure (éventuellement moyens) distincts l'un de l'autre.

Selon une variante préférentielle, lesdites première 202A et deuxième 202B parties courbes de la première surface articulaire 202 sont respectivement globalement assimilables à une première et à une deuxième fractions de surfaces (fictives) sensiblement tronconiques, lesdites première et deuxième fractions de surfaces étant de préférence respectivement issues d'un premier et d'un deuxième cônes fictifs, virtuels, présentant respectivement un premier et un deuxième axes de rotation. De manière avantageuse, la valeur du demi-angle à l'apex de chacun desdits premier et deuxième cônes fictifs est de 8°. Le choix de cette configuration particulière contribue à reproduire au mieux la cinématique naturelle de la cheville anatomique. Alternativement, lesdites première 202A et deuxième 202B parties courbes de la première surface articulaire 202 pourraient être respectivement globalement assimilables à une première et à une deuxième fractions de surfaces sensiblement cylindriques, lesdites première et deuxième fractions de surfaces étant de préférence respectivement issues d'un premier et d'un deuxième cylindres fictifs, présentant respectivement un premier et un deuxième axes de rotation. En d'autres termes, si ladite première surface articulaire 202 présente, comme introduit ci-avant, la forme générale moyenne d'une fraction de surface (fictive) sensiblement tronconique issue d'un cône fictif (ou cylindrique), cette forme générale moyenne est plus précisément définie, au sens de l'invention, par la combinaison d'au moins une première et une deuxième fractions distinctes de surfaces sensiblement tronconiques (ou cylindriques). Chacune de ces surfaces sensiblement tronconiques est préférentiellement orientée pour que sa grande base soit orientée en direction de la malléole externe et que sa petite base soit orientée en direction de la malléole interne du pied considéré. Ainsi, la première fraction de surface tronconique correspondant à la première partie courbe 202A présente un rayon de courbure qui varie, dans un sens latéro-médial de ladite deuxième direction B-B' moyenne, de manière décroissante entre un grand rayon R₁ de courbure et un petit rayon r₁ de courbure. La deuxième fraction de surface tronconique, correspondant à la deuxième partie courbe 202B, présente quant à elle un rayon de courbure qui varie, dans un sens latéro-médial de ladite deuxième direction B-B' moyenne, de manière décroissante entre un grand rayon R₂ de courbure et un petit rayon r₂ de courbure. Alternativement, dans le cas où lesdites première 202A et deuxième 202B parties courbes sont globalement assimilables à une première et à une deuxième fractions de surfaces sensiblement cylindriques, ces dernières pourront respectivement présenter un rayon de courbure R'₁, R'₂ qui est constant selon ladite deuxième direction B-B' moyenne.

Conformément à l'invention, lesdites première et deuxième courbures sont différentes, de sorte que l'une desdites première 202A et deuxième 202B parties courbes est donc plus courbée, c'est-à-dire plus fortement incurvée, que l'autre. Selon le mode de réalisation préférentiel, envisagé ci-avant, dans lequel lesdites première 202A et deuxième 202B parties courbes de la première surface articulaire 202 sont respectivement globalement assimilables à une première et à une deuxième fractions de surfaces (fictives) sensiblement tronconiques, une telle différence de courbure peut se traduire par le fait que la première fraction de surface tronconique présente un grand rayon R₁ de courbure et un petit rayon r₁ de courbure qui sont respectivement différents des grand rayon R₂ de courbure et petit rayon r₂ de courbure respectifs de la deuxième fraction de surface tronconique (R₁ ≠ R₂ et r₁ ≠ r₂). Selon la variante alternative dans laquelle lesdites première 202A et deuxième 202B parties courbes sont respectivement globalement assimilables à une première et à une deuxième fractions de surfaces sensiblement cylindriques, une telle différence de courbure pourra se traduire par le fait que la première fraction de surface cylindrique présente un rayon R'₁ de courbure différent du rayon de courbure R'₂ respectif de la deuxième fraction de surface cylindrique. Il en résulte que la première surface articulaire 202, définie par la face supérieure talaire 201, ne présente avantageusement pas la forme exacte et parfaite d'une portion de surface tronconique (ou cylindrique, comme envisagé ci-avant en alternative), mais qu'elle comporte au contraire une variation localisée particulière dans sa courbure générale moyenne selon ladite première direction A-A' moyenne. La cinématique générale de la prothèse 100 n'est alors pas définie par une unique rotation, mais par au moins deux rotations selon des rayons différents, correspondant à au moins deux configurations spatiales du pied relativement au tibia. Il est ainsi possible de générer un mouvement articulaire complexe, avec des axes de rotation différenciés de l'articulation prothétique entre une position de flexion plantaire et une position de flexion dorsale, et d'approcher de manière encore plus fidèle le comportement cinématique naturelle d'une cheville anatomique.

Dans le mode de réalisation préférentiel illustré aux figures, ladite première partie courbe 202A et ladite deuxième partie courbe 202B définissent (ou contribuent à définir au moins en partie) respectivement une portion antérieure et une portion postérieure de ladite première surface articulaire 202. A ce titre, ladite première partie courbe 202A s'étend préférentiellement entre le bord antérieur talaire 203 et le bord postérieur talaire 204 de la face supérieure talaire 201 du composant talaire 200, et de préférence encore, depuis ledit bord antérieur talaire 203 (ou au moins depuis le voisinage immédiat de ce dernier) en direction dudit bord postérieur talaire 204. Ladite deuxième partie courbe 202B s'étend alors respectivement entre ladite première partie courbe 202A et ledit bord postérieur talaire 204. De préférence encore, ladite deuxième partie courbe 202B est contigüe à ladite première partie courbe 202A et prolonge cette dernière jusqu'audit bord postérieur talaire 204 (ou au moins jusqu'au voisinage immédiat de ce dernier). La première partie courbe 202A et la deuxième partie courbe 202B sont donc alignées l'une derrière l'autre selon la première direction A-A' moyenne, la première partie courbe 202A (portion antérieure) étant agencée devant la deuxième partie courbe 202B (portion postérieure). Ainsi, lorsqu'elle est vue en coupe dans plan parallèle à la première direction A-A' moyenne et orthogonal à la deuxième direction B-B' moyenne (figure 10), ladite première surface articulaire 202 est décrite par au moins deux portions curvilignes distinctes et préférentiellement aboutées, formant chacune de préférence un arc, et reliant de préférence le bord antérieur talaire 203 au bord postérieur talaire 204. Ladite première partie courbe 202A correspond alors avantageusement à une partie de la première surface articulaire 202 avec laquelle coopérera la deuxième surface articulaire 403 du composant intermédiaire 400, en configuration de flexion dorsale du pied du patient (figure 5), tandis que ladite deuxième partie courbe 202 correspond à une autre partie de la première surface articulaire 202 avec laquelle coopérera ladite deuxième surface articulaire 403 du composant intermédiaire 400, en configuration de flexion plantaire du pied du patient (figure 3).

Avantageusement, lesdites première et deuxième courbures, ainsi que le positionnement relatif desdits premier et deuxième axes de rotation, seront choisis de sorte à assurer une transition régulière et harmonieuse entre lesdites première 202A et deuxième 202B parties courbes de la première surface articulaire 202.

De préférence, lesdites première et deuxième fractions de surfaces sensiblement tronconiques se rejoignent dans un plan de contact incliné d'un angle compris entre 10° et 30°, et de préférence d'environ 20°, en direction dudit bord antérieur talaire 203 par rapport à un plan vertical contenant ledit deuxième axe de rotation, ledit plan de contact contenant lesdits premier et deuxième axes de rotation. En effet, il a été observé qu'on obtient ainsi un excellent compromis entre l'augmentation du débattement angulaire apporté et la stabilité intrinsèque de la prothèse.

De préférence, ladite première courbure de la première partie courbe 202A est supérieure à ladite deuxième courbure de la deuxième partie courbe 202B, c'est-à-dire que ladite première partie courbe 202A de la première surface articulaire 202 affecte, selon la première direction A-A' moyenne, une courbure plus importante que celle respective de ladite deuxième partie courbe 202B de la première surface articulaire 202 (R₁ < R₂ et r₁ < r₂, ou R'₁ < R'₂). Dans le cas préférentiel évoqué ci-avant où ladite première partie 202A et ladite deuxième partie 202B courbes définissent respectivement une portion antérieure et une portion postérieure de la première surface articulaire 202, ladite première surface articulaire 202 présente alors avantageusement une courbure plus prononcée dans sa portion antérieure que dans sa portion postérieure. Une telle configuration s'avère particulièrement intéressante, puisqu'elle permet d'autoriser pour le patient une flexion dorsale avec un débattement angulaire plus important, sans pour autant que le talus du patient ne soit de fait déporté dans un sens antéro-postérieur, c'est-à-dire vers l'arrière. Equipé de la prothèse 100 selon l'invention, le patient pourra ainsi plus facilement fléchir son pied, par exemple lors de la phase de la marche où le pied quitte le sol à la fin d'un pas, ou lorsqu'il cherche à gravir les marches d'un escalier.

De préférence, ladite première courbure est constante ou variable (selon la première direction A-A' moyenne), tandis que ladite deuxième courbure est constante (selon la première direction A-A' moyenne). Une telle première courbure variable peut avantageusement correspondre à un mode de réalisation particulier selon lequel la forme générale de la première partie courbe 202A est elle-même globalement définie par la combinaison d'une pluralité *n* de fractions distinctes de surfaces sensiblement tronconiques ou cylindriques (et non par la seule première fraction évoquée ci-avant), lesquelles fractions présenteraient des petits r*n* et grands R*n* rayons (ou des rayons R'*n*) de courbure différents (et donc des nièmes courbures différentes), avantageusement décroissants selon ladite première direction A-A' moyenne. Dans le cas préférentiel où la première partie courbe 202A définit une portion antérieure de la première surface articulaire 202 et où lesdites *n*ièmes courbures varient de manière croissante en direction du bord antérieur tibial 203, la mise en œuvre d'une telle première courbure variable permet avantageusement d'améliorer encore l'amplitude du débattement angulaire offert par la prothèse 100 en flexion dorsale.

Les efforts intra-articulaires, susceptibles de s'exercer à l'interface des première 202 et deuxième 403 surfaces articulaires, étant plus importants en phase de flexion plantaire qu'en phase de flexion dorsale, ladite deuxième partie courbe 202B est préférentiellement conçue et dimensionnée de sorte qu'elle présente avantageusement une superficie supérieure à la superficie respective de ladite première partie courbe 202A. On assure ainsi une meilleure reprise des efforts intra-articulaires en flexion plantaire, ce qui permet d'améliorer tant la stabilité que la durée de vie de la prothèse 100.

De préférence, la première surface articulaire 202 forme une surface bicondylaire prothétique, et comprend :
- une zone latérale talaire 210L, qui s'étend entre ledit bord antérieur talaire 203 et ledit bord postérieur talaire 204 selon ladite première direction A-A' moyenne, et entre ledit bord latéral talaire 205 et ledit bord médial talaire 206 selon ladite deuxième direction B-B' moyenne, et
- une zone médiale talaire 210M, qui s'étend entre ledit bord antérieur talaire 203 et ledit bord postérieur talaire 204 selon ladite première direction A-A' moyenne, et entre ladite zone latérale talaire 210L et ledit bord médial talaire 206 selon ladite deuxième direction B-B' moyenne.

De préférence encore, tel qu'illustré notamment aux figures 6 et 8, lesdites zone latérale talaire 210L et zone médiale talaire 210M s'étendent respectivement depuis ledit bord antérieur talaire 203 (ou au moins depuis le voisinage immédiat de ce dernier) jusqu'audit bord postérieur talaire 204 (ou au moins jusqu'au voisinage immédiat de ce dernier). Ladite zone latérale talaire 210L comprend alors une première région latérale 211L de ladite première partie courbe 202A et une deuxième région latérale 212L de ladite deuxième partie courbe 202B. Ladite zone médiale talaire 210M comprend respectivement une première région médiale 211M de ladite première partie courbe 202A et une deuxième région médiale 212M de ladite de deuxième partie courbe 202B. Tel qu'illustré aux figures, les deuxième région latérale 212L et deuxième région médiale 212M prolongent alors de préférence respectivement lesdites première région latérale 211L et première région médiale 211L, selon la première direction A-A' moyenne. Avantageusement, lesdites zone latérale talaire 210L et zone médiale talaire 210M forment ainsi respectivement des parties latérale et médiale de condyle prothétique continues selon la première direction A-A' moyenne.

De manière avantageuse, ladite première surface articulaire 202 comprend également une zone centrale talaire 210C, qui est intercalée entre ladite zone latérale talaire 210L et ladite zone médiale talaire 210M. Tel qu'illustré aux figures, ladite zone centrale talaire 210C s'étend, selon ladite première direction A-A' moyenne, depuis ledit bord antérieur talaire 203 (ou au moins depuis le voisinage immédiat de ce dernier) en direction du bord postérieur talaire 204, et comprend avantageusement une première région centrale 211C de ladite première partie courbe 202A. De manière particulièrement avantageuse, ladite zone centrale talaire 210C comprend également une deuxième région centrale 213C, courbe, qui prolonge ladite première région centrale 211C en direction du bord postérieur talaire 204. Cependant, ladite deuxième région centrale 213C ne constitue de préférence pas, en tant que telle, une région centrale de la deuxième partie courbe 202B. En effet, ladite deuxième région centrale 213C présente avantageusement une troisième courbure, selon la première direction A-A' moyenne, qui est différente de ladite deuxième courbure de la deuxième partie courbe 202B. Ladite deuxième partie courbe 202B est alors discontinue selon la deuxième direction B-B' moyenne. En l'espèce, et en particulier selon le mode de réalisation préférentiel illustré aux figures dans lequel la première courbure de la première partie courbe 202A est supérieure à la deuxième courbure de la deuxième partie courbe 202B, ladite troisième courbure de la deuxième région centrale 213C est avantageusement supérieure à ladite deuxième courbure. Ainsi, la deuxième région centrale 213C est donc plus fortement courbée que les deuxième région latérale 212L et deuxième région médiale 212M environnantes. Par exemple, ladite troisième courbure pourra être identique à ladite première courbure, la deuxième région centrale 213C s'inscrivant dans la continuité de la première région centrale 211C.

Tel que cela ressort notamment des figures 6 et 8, ladite deuxième région centrale 213C matérialise alors avantageusement une dépression centrale postérieure 214 dans l'enveloppe moyenne de ladite première surface articulaire 202, En outre, ladite zone centrale talaire 210C de la première surface articulaire 202 peut ne pas s'étendre jusqu'au bord postérieur talaire 204 de la surface supérieure talaire 201. L'espace ainsi formé entre ladite zone centrale talaire 210C et ledit bord postérieur talaire 204 peut être laissé libre ou, au contraire, être préférentiellement comblé par une portion complémentaire 215 de face supérieure talaire 201 pleine, et par exemple plane, de manière à éviter la formation d'une zone de coupe osseuse non recouverte.

Selon le mode de réalisation préférentiel illustré aux figures, ladite deuxième surface articulaire 403, qui est définie par la face inférieure intermédiaire 402 du composant intermédiaire 400, comprend avantageusement une zone latérale intermédiaire 416L, une zone médiale intermédiaire 416M, et de préférence également une zone centrale intermédiaire 416C, cette dernière étant intercalée entre les deux précédentes. S'étendant chacune entre lesdits deuxième bord antérieur intermédiaire 408 et deuxième bord postérieur intermédiaire 409 selon ladite cinquième direction E-E' moyenne, lesdites zone latérale intermédiaire 416L, zone médiale intermédiaire 416M et zone centrale intermédiaire 416C sont respectivement destinées à coopérer avec lesdites zone latérale talaire 210L, zone médiale talaire 210M et zone centrale talaire 210C de ladite première surface articulaire 202. De préférence, lesdites zone latérale intermédiaire 416L et zone médiale intermédiaire 416M présentent avantageusement, selon ladite cinquième direction E-E' moyenne, des courbures respectivement conjuguées aux courbures desdites deuxième région latérale 212L et deuxième région médiale 212M de la deuxième partie courbe 202B de ladite première surface articulaire 202. Ladite zone centrale intermédiaire 416C présente quant à elle avantageusement, selon ladite cinquième direction E-E' moyenne, une courbure conjuguée à la courbure de ladite première région centrale 211C de la première partie courbe 202A de ladite première surface articulaire 202.

De préférence, et tel que cela est notamment visible aux figures 6 à 8, ladite zone centrale talaire 210C présente une courbure convexe selon ladite deuxième direction B-B' moyenne, lesdites zone latérale talaire 210L et zone médiale talaire 210M présentant respectivement une courbure concave selon ladite deuxième direction B-B' moyenne. Réciproquement, la zone latérale intermédiaire 416L et la zone médiale intermédiaire 416M sont avantageusement convexes selon ladite sixième direction F-F' moyenne, tandis que la zone centrale intermédiaire 416C est concave selon cette même direction F-F' (figure 17). Ainsi, en position de flexion plantaire (figure 3) du pied du patient, lesdites zone latérale intermédiaire 416L et zone médiale intermédiaire 416M peuvent avantageusement reposer respectivement en contact surfacique contre lesdites deuxième région latérale 212L et deuxième région médiale 212M de la deuxième partie courbe 202B de la première surface articulaire 202. En position de flexion dorsale (figure 5), ladite zone centrale intermédiaire 416C peut avantageusement reposer en contact surfacique avec la première région centrale 211C de la première partie courbe 202A de la première surface articulaire 202. En revanche, lesdites zone latérale intermédiaire 416L et zone médiale intermédiaire 416M ne peuvent alors pas, de préférence, reposer en contact surfacique contre lesdites première région latérale 211L et première région médiale 211M de la première partie courbe 202A de la première surface articulaire 202, du fait de leurs courbures respectives différentes.

Ainsi, la coopération desdites première et deuxième surfaces articulaires n'est avantageusement pas parfaitement congruente. En position de flexion dorsale, le composant intermédiaire 400 reste avantageusement libre de glisser et basculer légèrement, en latéral ou en médial, autour d'une position d'équilibre. Dans le cas d'une bascule latérale, la zone latérale intermédiaire 416L de la deuxième surface articulaire 403 peut avantageusement venir en contact linéaire contre la première région latérale 211L de la première partie courbe 202A de la première surface articulaire 202 définie par la face supérieure talaire 201 du composant talaire 200. Réciproquement, dans le cas d'une bascule médiale, la zone médiale intermédiaire 416M de ladite deuxième surface articulaire 403 peut avantageusement venir en contact linéaire contre la première région médiale 211M de la première partie courbe 202A de la première surface articulaire 202. En outre, en position de flexion dorsale, le composant intermédiaire 400 présente alors avantageusement une certaine latitude, limitée, de rotation autour d'un axe vertical. En permettant ainsi au composant intermédiaire 400 de conserver une légère mobilité en translation selon une direction médio-latérale et en rotation selon un axe vertical, une telle configuration contribue avantageusement à limiter les risques de descellement des composants talaire 200 et tibial 300 sous l'effet des contraintes mécaniques qui s'exercent sur la prothèse 100, en usage normal de cette dernière. Egalement, une telle configuration permet avantageusement de compenser, dans une certaine mesure, un léger défaut de positionnement relatif des composants talaire 200 et tibial 300, ce qui contribue à faciliter la mise en place de la prothèse 100 par le chirurgien dans le corps du patient.

Etant donné que la zone centrale intermédiaire 416C présente de préférence, conformément à ce qui précède, une courbure supérieure à celles respectives des zone latérale intermédiaire 416L et zone médiale intermédiaire 416M, elle peut alors définir, à proximité du deuxième bord postérieur intermédiaire 409, une protrusion 417 qui fait saillie de la surface de la face inférieure intermédiaire 402 (figure 17). Afin d'éviter toute interaction mécanique entre ladite protrusion 417 et la première surface articulaire 202, ladite dépression centrale postérieure 214 (matérialisée par la deuxième région centrale 213C de la zone centrale talaire 210C, comme évoqué ci-avant) est avantageusement laissée vide. Ainsi, en position de flexion plantaire, ladite protrusion 417 peut avantageusement s'effacer dans ladite dépression centrale postérieure 214.

Selon une variante (non illustrée aux figures), ledit composant tibial 300 est conçu pour être mobile relativement audit composant intermédiaire 400, lesdits composants tibial 300 et intermédiaire 400 n'étant alors préférentiellement pas reliés entre eux mécaniquement. Dans ce cas, ladite face inférieure tibiale 302 et ladite face supérieure intermédiaire 401 sont de préférence sensiblement planes et lisses, de sorte à ce que le composant tibial 300 et le composant intermédiaire 400 puissent venir en contact surfacique plan l'un contre l'autre. En usage normal de la prothèse 100, le composant tibial 300 et le composant intermédiaire 400 sont ainsi en contact l'un contre l'autre, et sont mobiles l'un par rapport à l'autre selon trois degrés de liberté, à savoir selon une translation de direction moyenne antéro-postérieure, une translation de direction moyenne médio-latérale, et une rotation autour d'un axe orthogonal au plan de contact desdites face inférieure tibiale 302 et face supérieure intermédiaire 401 entre elles. Selon une autre variante, illustrée aux figures 1 à 5, ledit composant tibial 300 est conçu pour être fixé, c'est-à-dire solidarisé, audit composant intermédiaire 400, de sorte à supprimer avantageusement tout degré de liberté entre le composant tibial 300 et le composant intermédiaire 400. Une telle solidarisation de ces derniers permet d'améliorer avantageusement la stabilité de l'articulation prothétique.

De préférence, lesdits composant tibial 300 et composant intermédiaire 400 sont conçus pour être fixés l'un à l'autre à l'aide de premier et deuxième éléments de solidarisation complémentaires respectifs, de préférence encore selon un assemblage en queue d'aronde. Selon le mode de réalisation préférentiel illustré aux figures, la face inférieure tibiale 302 du composant tibial 300 est ainsi avantageusement pourvue d'une rainure 311, par exemple de section trapézoïdale, qui forme avantageusement ledit premier élément de solidarisation (ou organe femelle en queue d'aronde). La face supérieure intermédiaire 401 du composant intermédiaire 400 est réciproquement avantageusement pourvue d'un tenon 418, qui forme ledit deuxième élément de solidarisation (ou organe mâle en queue d'aronde). Tel qu'illustré en exemple aux figures 16 et 18, ledit tenon 418 fait saillie de la surface de ladite face supérieure intermédiaire 401 et est de forme et dimensions complémentaires de celle de ladite rainure 311. De préférence, ladite rainure 311 s'étend longitudinalement selon ladite troisième direction C-C' moyenne, depuis la face antérieure tibiale 309 du composant tibial 300, au niveau duquel elle débouche, en direction de la face postérieure tibiale 310, et ce sur au moins la moitié de la distance moyenne qui sépare lesdites face antérieure tibiale 309 et face postérieure tibiale 310 (figures 12 et 14). Avantageusement, ledit tenon 418 s'étend longitudinalement selon ladite cinquième direction E-E' moyenne, depuis la face antérieure intermédiaire 414 du composant intermédiaire 400, en direction de la face postérieure intermédiaire 415 de ce dernier. De préférence, la longueur dudit tenon 418 est inférieure à la longueur respective de ladite rainure 311 (figures 16 et 18). Ladite rainure 311 est ainsi avantageusement conçue et configurée pour recevoir à coulissement étroit ledit tenon 418, lesdites face inférieure tibiale 302 et face supérieure intermédiaire 401 étant alors maintenues plaquées l'une contre l'autre. Un tel assemblage relatif en queue d'aronde permet ainsi avantageusement de bloquer au moins toute translation médio-latérale et toute rotation du composant intermédiaire 400 relativement au composant tibial 300. Avantageusement, ledit tenon 418 pourra être pourvu, au niveau de son extrémité postérieure de pentes 419A, 419B latérales (ou de chanfreins) conçues pour faciliter, guider, l'introduction du tenon 418 dans la rainure 311 lors de l'assemblage relatif du composant tibial 300 et du composant intermédiaire 400.

Bien évidemment, une configuration inversée pourrait tout à fait être envisagée, ladite face inférieure tibiale 302 étant pourvue dudit tenon, ladite face supérieure intermédiaire 401 étant réciproquement pourvue de ladite rainure. Tout autre moyen de fixation réciproque convenable, mettant en œuvre des premier et deuxième moyens de solidarisation différents de ceux décrits ci-avant, pourrait également être mis en oeuvre. En outre, on pourra avantageusement prévoir que, pour différentes tailles de composant tibial 300 et de composant intermédiaire 400, les dimensions de ladite rainure 311 et dudit tenon 418 sont respectivement identiques. Il sera alors avantageusement possible de proposer au chirurgien un kit prothétique comprenant une gamme de composants talaires 200, tibiaux 300 et intermédiaires 400, respectivement de différentes tailles pour s'adapter à la morphologie du patient à traiter, tout en assurant la compatibilité entre un composant tibial 300 et un composant intermédiaire 400 qui correspondent à des prothèses de tailles différentes.

Dans le cas préférentiel d'un tel assemblage en queue d'aronde, lesdits composant tibial 300 et composant intermédiaire 400 comprennent en outre de préférence respectivement des premier et deuxième éléments de butée complémentaires conçus pour limiter ou bloquer la translation antéro-postérieure (c'est-à-dire de l'avant vers l'arrière) du composant intermédiaire 400 relativement au composant tibial 300, lesdits premier et deuxième éléments de butée étant avantageusement distincts desdits premier et deuxième éléments de solidarisation complémentaires. En d'autres termes, la limitation (et de préférence le blocage) de la translation antéro-postérieure n'est avantageusement pas assuré(e) (ou, tout du moins, pas de manière exclusive) par l'assemblage en queue d'aronde lui-même, mais par la coopération desdits premier et deuxième éléments de butée complémentaires. On évite ainsi avantageusement la reprise, par les seuls rainure 311 et tenon 418, des efforts mécaniques appliqués au composant tibial 300 en usage de la prothèse 100. De préférence, lesdits premier et deuxième éléments de butée comprennent respectivement un rebord 312 positionné au moins au niveau du bord postérieur tibial 304 de la face inférieure tibiale 302 du composant tibial 300, et un dégagement 420 ménagé au niveau de la face supérieure intermédiaire 401 du composant intermédiaire 400 (par exemple au niveau du premier bord postérieur intermédiaire 405) et de profil complémentaire à celui dudit rebord 312. Ledit dégagement 420 est ainsi conçu et configuré pour recevoir ledit rebord 312, lesdites face inférieure tibiale 302 et face supérieure intermédiaire 401 étant avantageusement en contact plan l'une contre l'autre. La coopération desdits rebord 312 et dégagement 420 vient ainsi limiter, et de préférence bloquer, la course antéro-postérieure du tenon 418 dans la rainure 311. Bien évidemment des éléments de butée de conception différente pourraient être mis en oeuvre.

Selon le mode de réalisation préférentiel illustré aux figures, ledit rebord 312 est positionné au niveau desdits bord postérieur tibial 304, bord latéral tibial 305 et bord médial tibial 306 de la face inférieure tibiale 302 du composant tibial 300, et s'étend avantageusement de manière sensiblement continue le long des bords 304, 305, 306 en question. Ledit rebord 312 présente préférentiellement une section rectangulaire, constante ou non. Réciproquement, ledit dégagement 420 est préférentiellement ménagé au niveau des premier bord postérieur intermédiaire 405, premier bord latéral intermédiaire 406 et premier bord médial intermédiaire 407 de la face supérieure intermédiaire 401 dudit composant intermédiaire 400, avantageusement de manière sensiblement continue le long des bords 405, 406, 407 en question.

La mise en œuvre de tels rebord 312 et dégagement 420 complémentaires avantageusement semi-périphériques, et leur coopération lorsque le composant intermédiaire 400 est fixé au composant tibial 300, permet ainsi non seulement de limiter le risque de cisaillement du tenon 418 dans ladite rainure 311, mais également de limiter avantageusement le risque de déformation du composant intermédiaire 400 par fluage sous l'effet de l'effort de compression exercé sur ce dernier par le composant tibial 300 en usage normal de la prothèse 100. La durée de vie du composant intermédiaire 400 s'en trouve ainsi sensiblement améliorée, ce qui permet de limiter le risque de devoir procéder à une opération chirurgicale ultérieure de remplacement de ce dernier.

De préférence, afin de bloquer également toute translation postéro-antérieure (c'est-à-dire de l'arrière vers l'avant) du composant intermédiaire 400 relativement au composant tibial 300, lorsque ces derniers sont fixés l'un à l'autre, ledit composant tibial 300 et composant intermédiaire 400 sont avantageusement respectivement pourvus de premier 313A, 313B et deuxième 421A, 421B moyens de verrouillage complémentaires. Tel qu'illustré aux figures 12 et 14, ledit premier moyen 313A, 313B de verrouillage peut, par exemple, prendre la forme d'encoches 313A, 313B en dent de scie ménagées dans le rebord 312 de la face inférieure tibiale 302, par exemple de manière symétrique au niveau du bord latéral tibial 305 et du bord médial tibial 306 dudit composant tibial 300. Réciproquement, ledit deuxième moyen 421A, 421 B de verrouillage peut, quant à lui, prendre la forme, par exemple, d'ergots 421A, 421B de formes complémentaires auxdites encoches 313A, 313B en dent de scie, agencés au niveau du dégagement 420 du composant intermédiaire 400 (figures 16 à 18). Le composant tibial 300 et le composant intermédiaire 400 peuvent ainsi être verrouillés l'un à l'autre par clipsage, la déformation élastique locale du matériau formant le composant intermédiaire 400 permettant l'engagement des ergots 421A, 421B de ce dernier dans les encoches 313A, 313B correspondantes du composant tibial 300. Bien évidemment, d'autres premier 313A, 313B et deuxième 421A, 421B moyens de verrouillage complémentaires convenables pourront être envisagés en lieu et place de ceux qui viennent d'être décrits.

De préférence, le composant tibial 300 est conçu pour être fixé de manière amovible au composant intermédiaire 400, afin notamment d'autoriser le remplacement du composant intermédiaire 400 en cas de dégradation de ce dernier (usure, déformation, etc.). Dans ce cas, les premier 313A, 313B et deuxième 421A, 421B moyens de verrouillage complémentaires seront conçus pour autoriser un verrouillage réversible du composant tibial 300 et du composant intermédiaire 400 entre eux. A ce titre, le composant intermédiaire 400 pourra, par exemple, être pourvu d'évidements 422A, 422B ménagés dans la face supérieure intermédiaire 401 au voisinage immédiat des ergots 421A, 421B formant ledit deuxième moyen 421A, 421B de verrouillage, et conçus pour recevoir l'extrémité d'un instrument externe (non représenté, par exemple un tournevis plat). L'action de cet instrument externe, par exemple par effet levier ou par mise en rotation, permettra avantageusement une déformation élastique locale du matériau formant le composant intermédiaire 400 et un découplage des encoches 313A, 313B et ergots 421A, 421B, autorisant ainsi la désolidarisation des composants talaire 300 et intermédiaire 400.

Avantageusement, le composant talaire 200 est doté au niveau de sa face inférieure talaire 207 d'au moins un moyen d'ancrage talaire 216A, conçu pour assurer l'ancrage du composant talaire 200 dans la masse osseuse du talus concerné. Faisant préférentiellement saillie de la face inférieure talaire 207 du composant talaire 200, ledit moyen d'ancrage talaire 216A est avantageusement destiné à venir se loger, lors de la mise en place du composant talaire 200 dans le corps du patient, dans un logement (ou trou) correspondant pratiqué au préalable (ou lors de la mise en place du composant talaire 200) par le chirurgien dans la masse osseuse du talus. Tel qu'illustré notamment aux figures 7 et 9, le composant talaire 200 est doté de deux moyens d'ancrage talaires 216A, 216B, formés par deux plots talaires 216A, 216B, par exemple cylindriques à tête arrondie, qui font saillie de la face inférieure talaire 207 du composant talaire 200. De préférence, lesdits plots talaires 216A, 216B s'étendent de manière inclinée, en direction du bord postérieur talaire 204. Bien évidemment, d'autres types, formes et configurations convenables de moyen d'ancrage talaire 216 pourront être envisagés.

Afin d'assurer une excellente résistance mécanique dudit moyen d'ancrage talaire 216A, en particulier en fatigue, ce dernier est préférentiellement relié à la face inférieure talaire 207, au niveau de sa base, par un congé talaire 217A, c'est-à-dire par une surface courbe, ou par une ou plusieurs nervures. Dans le mode de réalisation préférentiel illustré aux figures, chacun des moyens d'ancrage talaires 216A, 216B est ainsi avantageusement relié à la face inférieure talaire 207 par un congé talaire 217A, 217B respectif. Il a cependant été observé que la présence d'un tel congé 217A, 217B peut s'avérer gênante lors de la mise en place, par exemple par impaction, du composant talaire 200 dans le corps du patient, dans la mesure où un tel congé 217A, 217B fait alors saillie de la surface de la face inférieure talaire 207. En effet, les mèches de perçage habituellement disponibles pour réaliser des logements dans la masse osseuse ne permettent pas de réaliser facilement une fraisure qui pourrait recevoir le congé 217A, 217B. La mise en œuvre d'un tel congé 217A, 217B peut s'avérer particulièrement défavorable dans le cas, envisagé ci-avant, où la face inférieure talaire 207 est pourvue d'un revêtement de surface, puisque la présence de ce dernier tend, selon son épaisseur, à amplifier l'importance dudit congé 217A, 217B, et donc à gêner encore davantage la mise en place dudit composant talaire 200. Pour pallier cet inconvénient, ladite face inférieure talaire 207 est avantageusement pourvue d'au moins une cuvette talaire 218A, à partir du fond de laquelle ledit au moins un moyen d'ancrage talaire 216A fait saillie de ladite face inférieure talaire 207. Dans le mode de réalisation préférentiel illustré aux figures, ladite face inférieure talaire 207 est avantageusement pourvue d'une pluralité de cuvettes talaires 218A, 218B à partir du fond respectif desquelles lesdits plots talaires 216A, 216B font saillie de ladite face supérieure tibiale 301. Tel qu'illustré aux figures, ladite cuvette talaire 218A, 218B est avantageusement dimensionnée de sorte que ledit congé 217A, 217B est intégralement contenu dans ladite cuvette talaire 218A, 218B et ne fait donc pas saillie de la surface de la face inférieure talaire 207. Ainsi, ledit composant talaire 200 peut être mis en place de manière relativement simple et précise au niveau du talus, la face inférieure talaire 207 pouvant venir en contact surfacique parfait avec la zone du talus correspondante, et ce sans nécessiter d'outils spécifiques de perçage de la masse osseuse.

Avantageusement, le composant tibial 300 est doté au niveau de sa face supérieure tibiale 301 d'au moins un moyen d'ancrage tibial 314A, conçu pour assurer l'ancrage du composant tibial 300 dans la masse osseuse du tibia concerné. Faisant préférentiellement saillie de la face supérieure tibiale 301 du composant tibial 300, ledit moyen d'ancrage tibial 314A est avantageusement destiné à venir se loger, lors de la mise en place du composant tibial 300 dans le corps du patient, dans au moins un logement (ou trou) correspondant pratiqué au préalable (ou lors de la mise en place du composant tibial 300) par le chirurgien dans la masse osseuse du tibia.

Tel qu'illustré notamment aux figures 11 à 13 et 15, le composant tibial 300 est préférentiellement pourvu d'une pluralité de moyens d'ancrage tibiaux 314A, 314B, 314C, 314D, par exemple formés de deux plots tibiaux 314A, 314B cylindriques à tête arrondie, qui font saillie de ladite face supérieure tibiale 301 au voisinage du bord antérieur tibial 303, et de deux ailettes tibiales 314C, 314D, qui font saillie de ladite face supérieure tibiale 301 au voisinage du bord postérieur tibial 304. De préférence, lesdites ailettes tibiales 314C, 314D présentent des arêtes aigües, voire tranchantes, pour favoriser leur pénétration dans la masse osseuse du tibia. Avantageusement, lesdits plots tibiaux 314A, 314B et lesdites ailettes tibiales 314C, 314D s'étendent de manière inclinée en direction du bord postérieur tibial 304. L'angle d'inclinaison desdits plots tibiaux 314A, 314B et desdites ailettes tibiales 314C, 314D sera avantageusement choisi pour faciliter l'introduction du composant tibial 400 dans le corps du patient, et sa mise en place au niveau du tibia de ce dernier, en limitant la distraction articulaire nécessaire. Bien évidemment, d'autres types, formes et configurations convenables de moyen(s) d'ancrage tibial 314A, 314B, 314C, 314D pourront être envisagés.

De préférence, de manière sensiblement comparable à ce qui a été décrit ci-avant en lien avec ledit au moins moyen d'ancrage talaire 216A du composant talaire 200, ledit au moins un moyen d'ancrage tibial 314A est préférentiellement relié à la face supérieure tibiale 301, au niveau de sa base, par un congé tibial 315A. Ladite face supérieure tibiale 301 est alors avantageusement pourvue d'au moins une cuvette tibiale 316A, à partir du fond de laquelle ledit au moins un moyen de fixation tibial 314A fait saillie de ladite face supérieure tibiale 301. Dans le mode de réalisation préférentiel illustré aux figures, chacun des plots tibiaux 314A, 314B et chacune des ailettes tibiales 314C, 314D est ainsi avantageusement relié à la face supérieure tibiale 301 par un congé tibial 315A, 315B, 315A, 315B respectif. Ladite face supérieure tibiale 301 est alors avantageusement pourvue d'une pluralité de cuvettes tibiales 316A, 316B, 316A, 316B à partir du fond respectif desquelles lesdits plots tibiaux 314A, 314B et lesdites ailettes tibiales 314C, 314D font saillie de ladite face supérieure tibiale 301.

Tel qu'illustré aux figures 7 et 9, le bord latéral talaire 205 et le bord médial talaire 206 du composant talaire 200 sont de préférence respectivement pourvus d'une encoche latérale talaire 219L et d'une encoche médiale talaire 219M, avantageusement positionnées de manière symétrique au voisinage du bord antérieur talaire 203. Ladite encoche latérale talaire 219L et ladite encoche médiale talaire 219M sont avantageusement conçues pour coopérer avec un instrument externe (non illustré, par exemple un tournevis plat), pour permettre l'ablation du composant talaire 200, par exemple en cas de positionnement non satisfaisant ou en cas de complication médicale justifiant le retrait de tout ou partie de la prothèse 100. De manière analogue, le bord latéral tibial 305 et le bord médial tibial 306 du composant tibial 300, pourront avantageusement être respectivement pourvus d'une encoche latérale tibiale 317L et d'une encoche médiale tibiale 317M, avantageusement positionnées de manière symétrique au voisinage du bord antérieur tibial 303 (figures 11, 12 et 14).

## Revendications

1. Prothèse (100) de cheville, comprenant un composant talaire (200) qui comporte une face supérieure talaire (201) définissant une première surface articulaire (202) et qui s'étend entre un bord antérieur talaire (203) et un bord postérieur talaire (204) opposé selon une première direction (A-A') moyenne, ladite première surface articulaire (202) étant incurvée selon ladite première direction (A-A') moyenne, ladite première surface articulaire (202) comprenant une première partie courbe (202A) et une deuxième partie courbe (202B), qui s'étendent chacune selon ladite première direction (A-A') moyenne, ladite première partie courbe (202A) présentant une première courbure et ladite deuxième partie courbe (202B) présentant une deuxième courbure, ladite prothèse (100) étant **caractérisée en ce que** lesdites première (202A) et deuxième (202B) parties courbes définissent respectivement une portion antérieure et une portion postérieure de ladite première surface articulaire (202), ladite première courbure étant supérieure à ladite deuxième courbure.

2. Prothèse (100) selon la revendication précédente, **caractérisée en ce que** ladite première courbure est constante ou variable, tandis que ladite deuxième courbure est constante.

3. Prothèse (100) selon l'une quelconque des revendications précédentes, **caractérisée en ce que** ladite deuxième partie courbe (202B) présente une superficie supérieure à la superficie respective de ladite première partie courbe (202A).

4. Prothèse (100) selon l'une quelconque des revendications précédentes, **caractérisée en ce que** lesdites première (202A) et deuxième (202B) parties courbes de la première surface articulaire (202) sont respectivement globalement assimilables à une première et à une deuxième fractions de surfaces sensiblement tronconiques.

5. Prothèse (100) selon la revendication précédente, **caractérisée en ce que** lesdites première et deuxième fractions de surface sont respectivement issues d'un premier et d'un deuxième cônes fictifs, présentant respectivement un premier et un deuxième axes de rotation, lesdites première et deuxième fractions de surfaces se rejoignant dans un plan de contact incliné d'un angle compris entre 10° et 30°, et de préférence d'environ 20°, en direction dudit bord antérieur talaire (203) par rapport à un plan vertical contenant ledit deuxième axe de rotation, ledit plan de contact contenant lesdits premier et deuxième axes de rotation.

6. Prothèse (100) selon l'une quelconque des revendications précédentes, **caractérisée en ce que** ladite face supérieure talaire (201) s'étend entre un bord latéral talaire (205) et un bord médial talaire (206) opposé selon une deuxième direction (B-B') moyenne, et **en ce que** ladite première surface articulaire (202) comprend :
- une zone latérale talaire (210L), qui s'étend entre ledit bord antérieur talaire (203) et ledit bord postérieur talaire (204) selon ladite première direction (A-A') moyenne, et entre ledit bord latéral talaire (205) et ledit bord médial talaire (206) selon ladite deuxième direction (B-B') moyenne, ladite zone latérale talaire (210L) comprenant une première région latérale (211L) de ladite première partie courbe (202A) et une deuxième région latérale (212L) de ladite deuxième partie courbe (202B), et
- une zone médiale talaire (210M), qui s'étend entre ledit bord antérieur talaire (203) et ledit bord postérieur talaire (204) selon ladite première direction (A-A') moyenne, et entre ladite zone latérale talaire (210L) et ledit bord médial talaire (206) selon ladite deuxième direction (B-B') moyenne, ladite zone médiale talaire (210M) comprenant une première région médiale (211M) de ladite première partie courbe (202A) et une deuxième région médiale (212M) de ladite de deuxième partie courbe (202B).

7. Prothèse (100) selon la revendication précédente, **caractérisée en ce que** ladite première surface articulaire (202) comprend également une zone centrale talaire (210C), qui est intercalée entre ladite zone latérale talaire (210L) et ladite zone médiale talaire (210M), et qui comprend une première région centrale (211C) de ladite première partie courbe (202A) et une deuxième région centrale (213C) qui prolonge ladite première région centrale (211C) et présente une troisième courbure qui est supérieure à ladite deuxième courbure.

8. Prothèse (100) selon la revendication précédente, **caractérisée en ce que** ladite zone centrale talaire (210C) présente une courbure convexe selon ladite deuxième direction (B-B') moyenne, lesdites zone latérale talaire (210L) et zone médiale talaire (210M) présentant respectivement une courbure concave selon ladite deuxième direction (B-B') moyenne.

9. Prothèse (100) selon l'une quelconque des revendications précédentes, **caractérisée en ce qu'**elle comprend un composant tibial (300), qui comporte une face supérieure tibiale (301) et une face inférieure tibiale (302) opposée, ladite face inférieure tibiale (302) s'étendant d'une part entre un bord antérieur tibial (303) et un bord postérieur tibial (304) opposé et, d'autre part, entre un bord latéral tibial (305) et un bord médial tibial (306) opposé.

10. Prothèse (100) selon la revendication précédente, **caractérisée en ce qu'**elle comprend un composant intermédiaire (400), qui est conçu pour être intercalé entre ledit composant talaire (200) et ledit composant tibial (300), ledit composant intermédiaire (400) comprenant une face supérieure intermédiaire (401) et une face inférieure intermédiaire (402) opposée, ladite face inférieure intermédiaire (402) définissant une deuxième surface articulaire (403) conçue pour coopérer avec ladite première surface articulaire (202).

11. Prothèse (100) selon la revendication précédente et l'une quelconque des revendications 7 et 8, **caractérisée en ce que** ladite deuxième surface articulaire (403) comprend une zone latérale intermédiaire (416L), une zone médiale intermédiaire (416M) et une zone centrale intermédiaire (416C), respectivement destinées à coopérer avec lesdites zone latérale talaire (210L), zone médiale talaire (210M) et zone centrale talaire (210C) de ladite première surface articulaire (202), lesdites zone latérale intermédiaire (416L) et zone médiale intermédiaire (416M) présentant des courbures respectivement conjuguées aux courbures desdites deuxième région latérale (212L) et deuxième région médiale (212M) de la deuxième partie courbe (202B) de ladite première surface articulaire (202), tandis que ladite zone centrale intermédiaire (416C) présente une courbure conjuguée à la courbure de ladite première région centrale (211C) de la première partie courbe (202A) de ladite première surface articulaire (202).

12. Prothèse (100) selon l'une quelconque des revendications 10 et 11, **caractérisée en ce que** ledit composant tibial (300) est conçu pour être mobile relativement audit composant intermédiaire (400).

13. Prothèse (100) selon l'une quelconque des revendications 10 et 11, **caractérisée en ce que** ledit composant tibial (300) est conçu pour être fixé, de préférence de manière amovible, audit composant intermédiaire (400).

14. Prothèse (100) selon la revendication précédente, **caractérisée en ce que** lesdits composant tibial (300) et composant intermédiaire (400) sont conçus pour être fixés l'un à l'autre à l'aide de premier et deuxième éléments de solidarisation complémentaires respectifs selon un assemblage en queue d'aronde.

15. Prothèse (100) selon la revendication précédente, **caractérisée en ce que** lesdits composant tibial (300) et composant intermédiaire (400) comprennent en outre respectivement des premier et deuxième éléments de butée complémentaires conçus pour limiter ou bloquer la translation antéro-postérieure du composant intermédiaire (400) relativement au composant tibial (300), lesdits premier et deuxième éléments de butée étant distincts desdits premier et deuxième éléments de solidarisation complémentaires.

## Patentansprüche

1. Knöchelprothese (100), die ein Sprungbeinbauteil (200) umfasst, das eine obere Sprungbeinfläche (201) umfasst, die eine erste Gelenkoberfläche (202) definiert, und die sich zwischen einem vorderen Sprungbeinrand (203) und einem hinteren Sprungbeinrand (204), der entlang einer ersten mittleren Richtung (A-A') entgegengesetzt ist, erstreckt, wobei die erste Gelenkoberfläche (202) entlang der ersten mittleren Richtung (A-A') gewölbt ist, wobei die erste Gelenkoberfläche (202) einen ersten gewölbten Teil (202A) und einen zweiten gewölbten Teil (202B) umfasst, die sich jeweils entlang der ersten mittleren Richtung (A-A') erstrecken, wobei der erste gewölbte Teil (202A) eine erste Wölbung aufweist, und der zweite gewölbte Teil (202B) eine zweite Wölbung aufweist, wobei die Prothese (100) **dadurch gekennzeichnet ist, dass** der erste (202A) und der zweite (202B) gewölbte Teil jeweils einen vorderen Abschnitt und einen hinteren Abschnitt der ersten Gelenkoberfläche (202) definieren, wobei die erste Wölbung größer ist als die zweite Wölbung.

2. Prothese (100) nach dem vorstehenden Anspruch, **dadurch gekennzeichnet, dass** die erste Wölbung konstant oder variabel ist, während die zweite Wölbung konstant ist.

3. Prothese (100) nach einem der vorstehenden Ansprüche, **dadurch gekennzeichnet, dass** der zweite gewölbte Teil (202B) eine Oberfläche aufweist, die größer ist als die jeweilige Oberfläche des ersten gewölbten Teils (202A).

4. Prothese (100) nach einem der vorstehenden Ansprüche, **dadurch gekennzeichnet, dass** der erste (202A) und der zweite (202B) gewölbte Teil der ersten Gelenkoberfläche (202) jeweils insgesamt mit einem ersten und einem zweiten Bruchteil von Oberflächen, die im Wesentlichen kegelstumpfförmig sind, gleichstellbar sind.

5. Prothese (100) nach dem vorstehenden Anspruch, **dadurch gekennzeichnet, dass** der erste und der zweite Oberflächenbruchteil jeweils aus einem ersten und einem zweiten fiktiven Konus hervorgehen, die jeweils eine erste und eine zweite Rotationsachse aufweisen, wobei der erste und der zweite Oberflächenbruchteil in einer Berührungsebene zusammentreffen, die um einen Winkel zwischen 10° und 30° und vorzugsweise etwa 20° in Richtung des vorderen Sprungbeinrands (203) in Bezug auf eine vertikale Ebene geneigt ist, die die zweite Rotationsachse enthält, wobei die Berührungsebene die erste und die zweite Rotationsachse enthält.

6. Prothese (100) nach einem der vorstehenden Ansprüche, **dadurch gekennzeichnet, dass** sich die Sprungbeinfläche (201) zwischen einem seitlichen Sprungbeinrand (205) und einem medialen Sprungbeinrand (206), der entlang einer zweiten mittleren Richtung (B-B') entgegengesetzt ist, erstreckt, und dass die erste Gelenkoberfläche (202) Folgendes umfasst:
- eine seitliche Sprungbeinzone (210L), die sich zwischen dem vorderen Sprungbeinrand (203) und dem hinteren Sprungbeinrand (204) entlang der ersten mittleren Richtung (A-A'), und zwischen dem seitlichen Sprungbeinrand (205) und dem medialen Sprungbeinrand (206) entlang der zweiten mittleren Richtung (B-B') erstreckt, wobei die seitliche Sprungbeinzone (210L) einen ersten seitlichen Bereich (211L) des ersten gewölbten Teils (202A) und einen zweiten seitlichen Bereich (212L) des zweiten gewölbten Teils (202B) umfasst, und
- eine mediale Sprungbeinzone (210M), die sich zwischen dem vorderen Sprungbeinrand (203) und dem hinteren Sprungbeinrand (204) entlang der ersten mittleren Richtung (A-A') erstreckt, und zwischen dem seitlichen Sprungbeinrand (210L) und dem medialen Sprungbeinrand (206) entlang der zweiten mittleren Richtung (B-B'), wobei die mediale Sprungbeinzone (210M) einen ersten medialen Bereich (211M) des ersten gewölbten Teils (202A) und einen zweiten medialen Bereich (212M) des zweiten gewölbten Teils (202B) umfasst.

7. Prothese (100) nach dem vorstehenden Anspruch, **dadurch gekennzeichnet, dass** die erste Gelenkoberfläche (202) auch eine zentrale Sprungbeinzone (210C) umfasst, die zwischen die seitliche Sprungbeinzone (210L) und die mediale Sprungbeinzone (210M) eingefügt ist, und die einen ersten zentralen Bereich (211C) des ersten gewölbten Teils (202A) und einen zweiten zentralen Bereich (213C) umfasst, der den ersten zentralen Bereich (211C) verlängert und eine dritte Wölbung aufweist, die größer ist als die zweite Wölbung.

8. Prothese (100) nach dem vorstehenden Anspruch, **dadurch gekennzeichnet, dass** die zentrale Sprungbeinzone (210C) eine konvexe Wölbung entlang der zweiten mittleren Richtung (B-B') aufweist, wobei die seitliche Sprungbeinzone (210L) und die mediale Sprungbeinzone (210M) jeweils eine konkave Wölbung entlang der zweiten mittleren Richtung (B-B') aufweisen.

9. Prothese (100) nach einem der vorstehenden Ansprüche, **dadurch gekennzeichnet, dass** sie ein Tibiabauteil (300) umfasst, das eine obere Tibiafläche (301) und eine entgegengesetzte untere Tibiafläche (302) umfasst, wobei sich die untere Tibiafläche (302) einerseits zwischen einem vorderen Tibiarand (303) und einem entgegengesetzten hinteren Tibiarand (304) erstreckt, und andererseits zwischen einem seitlichen Tibiarand (305) und einem entgegengesetzten medialen Tibiarand (306) erstreckt.

10. Prothese (100) nach dem vorstehenden Anspruch, **dadurch gekennzeichnet, dass** sie ein Zwischenbauteil (400) umfasst, das konzipiert ist, um zwischen dem Sprungbeinbauteil (200) und dem Tibiabauteil (300) eingefügt zu sein, wobei das Zwischenbauteil (400) eine obere Zwischenfläche (401) und eine entgegengesetzte untere Zwischenfläche (402) umfasst, wobei die untere Zwischenfläche (402) eine zweite Gelenkoberfläche (403) definiert, die konzipiert ist, um mit der ersten Gelenkoberfläche (202) zusammenzuwirken.

11. Prothese (100) nach dem vorstehenden Anspruch und einem der Ansprüche 7 und 8, **dadurch gekennzeichnet, dass** die zweite Gelenkoberfläche (403) eine seitliche Zwischenzone (416L), eine mediale Zwischenzone (416M) und eine zentrale Zwischenzone (416C) umfasst, die jeweils dazu bestimmt sind, mit der seitlichen Sprungbeinzone (210L), der medialen Sprungbeinzone (210M) und einer zentralen Sprungbeinzone (210C) der ersten Gelenkoberfläche (202) zusammenzuwirken, wobei die seitliche Zwischenzone (416L) und die mediale Zwischenzone (416M) jeweilige Wölbungen aufweisen, die mit den Wölbungen des zweiten seitlichen Bereichs (212L) und des zweiten medialen Bereichs (212M) des zweiten gewölbten Teils (202B) der ersten Gelenkoberfläche (202) konjugiert sind, während die zentrale Zwischenzone (416C) eine Wölbung aufweist, die mit der Wölbung der ersten zentralen Zone (211C) des ersten gewölbten Teils (202A) der ersten Gelenkoberfläche (202) konjugiert ist.

12. Prothese (100) nach einem der Ansprüche 10 und 11, **dadurch gekennzeichnet, dass** das Tibiabauteil (300) konzipiert ist, um in Bezug auf das Zwischenbauteil (400) beweglich zu sein.

13. Prothese (100) nach einem der Ansprüche 10 und 11, **dadurch gekennzeichnet, dass** das Tibiabauteil (300) konzipiert ist, um, vorzugsweise auf abnehmbare Art, an dem Zwischenbauteil (400) befestigt zu sein.

14. Prothese (100) nach dem vorstehenden Anspruch, **dadurch gekennzeichnet, dass** das Tibiabauteil (300) und das Zwischenbauteil (400) konzipiert sind, um aneinander mit Hilfe eines ersten und eines zweiten Elements zur festen Verbindung, die gemäß einer Schwalbenschwanzverbindung zueinander komplementär sind, befestigt zu sein.

15. Prothese (100) nach dem vorstehenden Anspruch, **dadurch gekennzeichnet, dass** das Tibiabauteil (300) und das Zwischenbauteil (400) außerdem jeweils ein erstes und ein zweites komplementäres Anschlagelement umfassen, die konzipiert sind, um die antero-posteriore Verschiebung des Zwischenbauteils (400) in Bezug auf das Tibiabauteil (300) einzuschränken oder zu blockieren, wobei das erste und das zweite Anschlagelement von dem ersten und dem zweiten komplementären Element zur festen Verbindung unterschiedlich sind.

## Claims

1. An ankle prosthesis (100) comprising a talar component (200) which includes a talar upper face (201) defining a first articular surface (202) and which extends between a talar anterior edge (203) and an opposite talar posterior edge (204) according to a first average direction (A-A'), said first articular surface (202) being curved according to said first average direction (A-A'), said first articular surface (202) comprising a first curved portion (202A) and a second curved portion (202B), each extending according to said first average direction (A-A'), said first curved portion (202A) having a first curvature and said second curved portion (202B) having a second curvature, said prosthesis (100) being **characterized in that** said first curved portion (202A) and said second curved portion (202B) define respectively an anterior portion and a posterior portion of said first articular surface (202), said first curvature being greater than said second curvature.

2. The prosthesis (100) according to the preceding claim, **characterized in that** said first curvature is constant or variable, whereas said second curvature is constant.

3. The prosthesis (100) according to any one of the preceding claims, **characterized in that** said second curved portion (202B) has a surface area larger than the respective surface area of said first curved portion (202A).

4. The prosthesis (100) according to any one of the preceding claims, **characterized in that** said first (202A) and second (202B) curved portions of the first articular surface (202) are respectively generally similar to first and second substantially frustoconical surface fractions.

5. The prosthesis (100) according to the preceding claim, **characterized in that** said first and second surface fractions are respectively derived from first and second fictional cones, having respectively first and second axes of rotation, said first and second surface fractions joining together in a contact plane inclined at an angle comprised between 10° and 30°, and preferably about 20°, towards said talar anterior edge (203) relative to a vertical plane containing said second axis of rotation, said contact plane containing said first and second axes of rotation.

6. The prosthesis (100) according to any one of the preceding claims, **characterized in that** said talar upper face (201) extends between a talar lateral edge (205) and an opposite talar medial edge (206) according to a second average direction (B-B'), and **in that** said first articular surface (202) comprises:
- a talar lateral area (210L), which extends between said talar anterior edge (203) and said talar posterior edge (204) according to said first average direction (A-A'), and between said talar lateral edge (205) and said talar medial edge (206) according to said second average direction (B-B'), said talar lateral area (210L) comprising a first lateral region (211L) of said first curved portion (202A) and a second lateral region (212L) of said second curved portion (202B), and
- a talar medial area (210M), which extends between said talar anterior edge (203) and said talar posterior edge (204) according to said first average direction (A-A'), and between said talar lateral area (210L) and said talar medial edge (206) according to said second average direction (B-B'), said talar medial area (210M) comprising a first medial region (211M) of said first curved portion (202A) and a second medial region (212M) of said second curved portion (202B).

7. The prosthesis (100) according to the preceding claim, **characterized in that** said first articular surface (202) also comprises a talar central area (210C), which is interposed between said talar lateral area (210L) and said talar medial area (210M), and which comprises a first central region (211C) of said first curved portion (202A) and a second central region (213C) which extends said first central region (211C) and has a third curvature which is greater than said second curvature.

8. The prosthesis (100) according to the preceding claim, **characterized in that** said talar central area (210C) has a convex curvature according to said second average direction (B-B'), said talar lateral area (210L) and talar medial area (210M) having respectively a concave curvature according to said second average direction (B-B').

9. The prosthesis (100) according to any one of the preceding claims, **characterized in that** it comprises a tibial component (300), which includes a tibial upper face (301) and an opposite tibial lower face (302), said tibial lower face (302) extending, on the one hand, between a tibial anterior edge (303) and an opposite tibial posterior edge (304) and, on the other hand, between a tibial lateral edge (305) and an opposite tibial medial edge (306).

10. The prosthesis (100) according to the preceding claim, **characterized in that** it comprises an intermediate component (400), which is designed to be interposed between said talar component (200) and said tibial component (300), said intermediate component (400) comprising an intermediate upper face (401) and an opposite intermediate lower face (402), said intermediate lower face (402) defining a second articular surface (403) designed to cooperate with said first articular surface (202).

11. The prosthesis (100) according to the preceding claim and any one of claims 7 and 8, **characterized in that** said second articular surface (403) comprises an intermediate lateral area (416L), an intermediate medial area (416M) and an intermediate central area (416C), respectively intended to cooperate with said talar lateral area (210L), talar medial area (210M) and talar central area (210C) of said first articular surface (202), said intermediate lateral area (416L) and intermediate medial area (416M) having curvatures respectively matching with the curvatures of said second lateral region (212L) and second medial region (212M) of the second curved portion (202B) of said first articular surface (202), whereas said intermediate central area (416C) has a curvature matching with the curvature of said first central region (211C) of the first curved portion (202A) of said first articular surface (202).

12. The prosthesis (100) according to any one of claims 10 and 11, **characterized in that** said tibial component (300) is designed to be movable relative to said intermediate component (400).

13. The prosthesis (100) according to any one of claims 10 and 11, **characterized in that** said tibial component (300) is designed to be fastened, preferably removably, to said intermediate component (400).

14. The prosthesis (100) according to the preceding claim, **characterized in that** said tibial component (300) and intermediate component (400) are designed to be fastened to each other by means of respective first and second securing elements according to a dovetail assembly.

15. The prosthesis (100) according to the preceding claim, **characterized in that** said tibial component (300) and intermediate component (400) further comprise respectively first and second complementary stop elements designed to limit or block the antero-posterior translation of the intermediate component (400) relative to the tibial component (300), said first and second stop elements being distinct from said first and second complementary securing elements.
